# EUROPÄISCHE PATENTSCHRIFT

(11) **EP 2 572 629 B1**
(45) Veröffentlichungstag und Bekanntmachung des Hinweises auf die Patenterteilung: **21.05.2014**
(21) Anmeldenummer: 12185500.1
(22) Anmeldetag: 21.09.2012
(51) Int. Cl.: A61B 3/13, A61F 9/008, G02B 21/00

(54) **Anzeigevorrichtung und Anzeigeverfahren**
Display device and display method
Dispositif d'affichage et procédé d'affichage

(30) Priorität: 23.09.2011 DE 102011083354
(43) Veröffentlichungstag der Anmeldung: 27.03.2013
(73) Patentinhaber: Carl Zeiss Sports Optics GmbH, 35576 Wetzlar (DE)
(72) Erfinder: Bublitz, Daniel, 07646 Rausdorf (DE); Nieten, Christoph, 07743 Jena (DE); Geißler, Enrico, 07749 Jena (DE); Mohr, Thomas, 07745 Jena (DE); Högele, Artur, 73447 Oberkkochen (DE)
(74) Vertreter: Geyer, Fehners & Partner

(56) Entgegenhaltungen:
- US-A1- 2003 218 755

## Beschreibung

Die vorliegende Erfindung betrifft eine Anzeigevorrichtung gemäß dem Oberbegriff des Anspruches 1 sowie ein Anzeigeverfahren gemäß dem Oberbegriff des Anspruches 12.

Eine solche Anzeigevorrichtung kann z.B. als Lichtmikroskop, Operationsmikroskop, Spektiv, Spaltlampe oder Funduskamera ausgebildet sein, wobei mittels dem Bildaufnahmemodul das mit dem Beobachtungsmodul für den Benutzer zur Betrachtung abgebildete Objekt gleichzeitig aufgenommen wird. Diese Aufnahme wird z.B. zu Dokumentationszwecken und/oder für eine spätere Auswertung und Betrachtung des Objektes durchgeführt.

Weist das Auge des Benutzers eine Fehlsichtigkeit auf, so liegen die erste und zweite Fokusebene nicht übereinander. Dies hat zur Folge, daß das Bildaufnahmemodul unscharfe Bilder liefert im Vergleich zu dem, was der Benutzer sieht. Aus diesem Grunde ist häufig ein sogenannter Dioptrienausgleich vorgesehen, den der Benutzer einstellen kann, um eine Abstimmung zwischen den beiden Fokusebenen zu ermöglich. Es hat sich jedoch gezeigt, daß der Benutzer den Dioptrienausgleich häufig nicht korrekt einstellt, so daß in der Praxis häufig unscharfe Bildaufnahmen erzeugt werden.

Darüber hinaus hat der Benutzer bei dem Beobachtungsmodul aufgrund des rein optischen Einblick die Möglichkeit, durch eine Akkommodation des Auges die zweite Fokusebene zu verschieben. Auf diese Weise kann er sich z.B. einen dreidimensionalen Überblick über das Objekt verschaffen. Diese Möglichkeit steht dem Bildaufnahmemodul nicht offen, so daß die Aufnahmen des Bildaufnahmemoduls nicht dem entsprechen, was der Benutzer durch das rein optische Beobachtungsmodul gesehen hat.

Wenn das Bildaufnahmemodul zur Dokumentation bei einem Licht- oder Operationsmikroskop dient, führt es dazu, daß die dokumentierten Aufnahmen möglicherweise nicht die entscheidenden Details zeigen, da der Benutzer sein Auge anders fokussiert hat als das Bildaufnahmemodul.

Wenn das Bildaufnahmemodul einen Zweibeobachter ein digitales Kamerabild zur Verfügung stellt, sieht der Zweibeobachter nicht das Gleiche wie der Benutzer der Anzeigevorrichtung, der den rein optischen Beobachtungskanal verwendet.

Bei einem Fotografieren mit einem Spektiv sind die Aufnahmen unscharf gegenüber der Szene, die der Benutzer bei der Einstellung des Beobachtungsmoduls gewählt hat. Dies kann kritisch sein für Naturfotografen und Jäger aber auch bei militärischen Anwendungen oder bei der Grenzraumüberwachung.

Bei der Dokumentation bei Spaltlampen und Funduskameras zeigen die dokumentierten Aufnahmen möglicherweise auch nicht die entscheidenden Details, da der Benutzer (beispielsweise der Augenarzt) sein Auge anders fokussiert hat als das Bildaufnahmemodul. Dies führt zu dem Nachteil, daß sich die Pupille des Patienten schließt, so daß erst nach etlichen Minuten die Aufnahme wiederholt werden kann.

Ausgehend hiervon ist es Aufgabe der Erfindung, eine Anzeigevorrichtung der eingangs genannten Art sowie ein Anzeigeverfahren der eingangs genannten Art so weiterzubilden, daß die beschriebenen Schwierigkeiten so gut wie vollständig behoben werden.

Die Aufgabe wird bei einer Anzeigevorrichtung der eingangs genannten Art dadurch gelöst, daß ein Meßmodul zum Messen des Akkommodationszustandes des Auges vorgesehen ist und eine Steuereinheit vorgesehen ist, die in Abhängigkeit des gemessenen Akkommodationszustandes die Lage der ersten Fokusebene so einstellt, daß sie mit der zweiten Fokusebene zusammenfällt.

Somit wird erfindungsgemäß die Lage der ersten Fokusebene der Lage der zweiten Fokusebene, die sich durch Änderung des Akkommodationszustandes des Auges des Benutzers ändern kann, nachgeführt, so daß stets das aufgenommen wird, was der Benutzer scharf über das Beobachtungsmodul wahrnimmt.

Das Beobachtungsmodul ist bevorzugt als rein optisches Modul ausgebildet.

Anders gesagt, wird erfindungsgemäß die Lage der Fokusebene des digitalen Kanals (Bildaufnahmemodul) der Anzeigevorrichtung der Lage der zweiten Fokusebene des rein optischen Kanals (Beobachtungsmodul) nachgeführt.

Bei der erfindungsgemäßen Anzeigevorrichtung kann das Bildaufnahmemodul eine erste Optik zum Abbilden des Objektes auf den Bildsensor aufweisen. Bevorzugt wird noch zumindest ein Teil der Optik des Beobachtungsmoduls für die Abbildung auf den Bildsensor genutzt. Wenn das Beobachtungsmodul zumindest ein Objektiv aufweist, kann das Objektiv auch vom Bildaufnahmemodul verwendet werden.

Bei der erfindungsgemäßen Anzeigevorrichtung kann die Brechkraft der ersten Optik geändert werden, um die Lage der ersten Fokusebene einzustellen. Dies kann beispielsweise durch zumindest ein Element mit variabler Brechkraft und/oder durch mehrere optische Elemente, deren Lage zueinander verändert werden, realisiert werden.

Ferner kann zusätzlich oder alternativ zur Änderung der Brechkraft der ersten Optik der Abstand zwischen der ersten Optik und dem Bildsensor geändert werden, um die Lage der ersten Fokusebene einzustellen.

Bei der erfindungsgemäßen Anzeigevorrichtung kann das Meßmodul den Akkommodationszustand des Auges konfokal messen. Insbesondere kann das Meßmodul zur Messung des Akkommodationszustandes des Auges Infrarotstrahlung (bevorzugt mit einer Wellenlänge aus dem Bereich von 800 bis 1.060 nm) verwenden. Das Meßmodul kann den Akkommodationszustand z.B. laufend oder periodisch messen.

Ferner kann das Meßmodul eine Lichtquelle, die einen Lichtstrahl abgibt, ein optisches System, das den Lichtstrahl über das Beobachtungsmodul in das Auge des Benutzers führt, und einen Detektor aufweisen, wobei der am Augenhintergrund des Auges reflektierte Lichtstrahl über das Beobachtungsmodul und das optische System auf den Detektor gerichtet wird, der ein Detektorsignal abgibt, das zur Messung des Akkommodationszustandes genutzt wird.

Ferner kann bei der erfindungsgemäßen Anzeigevorrichtung das Bildaufnahmemodul und das Meßmodul gleichzeitig bewegt werden, wobei durch die Bewegung des Meßmoduls der Akkommodationszustand des Auges gemessen wird und durch die Bewegung des Bildaufnahmemoduls die Lage der ersten Fokusebene eingestellt wird. Insbesondere kann die Bewegung des Bildaufnahmemoduls in Abhängigkeit der Bewegung des Meßmoduls durchgeführt werden. So können das Bildaufnahmemodul und das Meßmodul z.B. mechanisch miteinander gekoppelt sein, so daß das Bildaufnahmemodul und das Meßmodul nur gleichzeitig bewegt werden können.

Die erfindungsgemäße Anzeigevorrichtung kann als Mikroskop (z.B. Lichtmikroskop, Operationsmikroskop), als Spektiv, als Spaltlampe oder als Funduskamera ausgebildet sein.

Die Aufgabe wird ferner bei einem Anzeigeverfahren der eingangs genannten Art dadurch gelöst, daß der Akkommodationszustand des Auges gemessen und in Abhängigkeit des gemessenen Akkommodationszustandes die Lage der ersten Fokusebene so eingestellt wird, daß sie mit der zweiten Fokusebene zusammenfällt.

Das erfindungsgemäße Anzeigeverfahren kann die Schritte aufweisen, die im Zusammenhang mit der erfindungsgemäßen Anzeigevorrichtung (einschließlich ihrer Weiterbildungen sowie der noch nachfolgenden Beschreibung von Ausführungsbeispielen) angegeben sind.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Ansicht einer ersten Ausführungsform der erfindungsgemäßen Anzeigevorrichtung;
- Fig. 2: eine Darstellung des optischen Aufbaus des Meßmoduls 8 von Fig. 1, und
- Fig. 3: eine schematische Darstellung einer weiteren Ausführungsform der erfindungsgemäßen Anzeigevorrichtung.

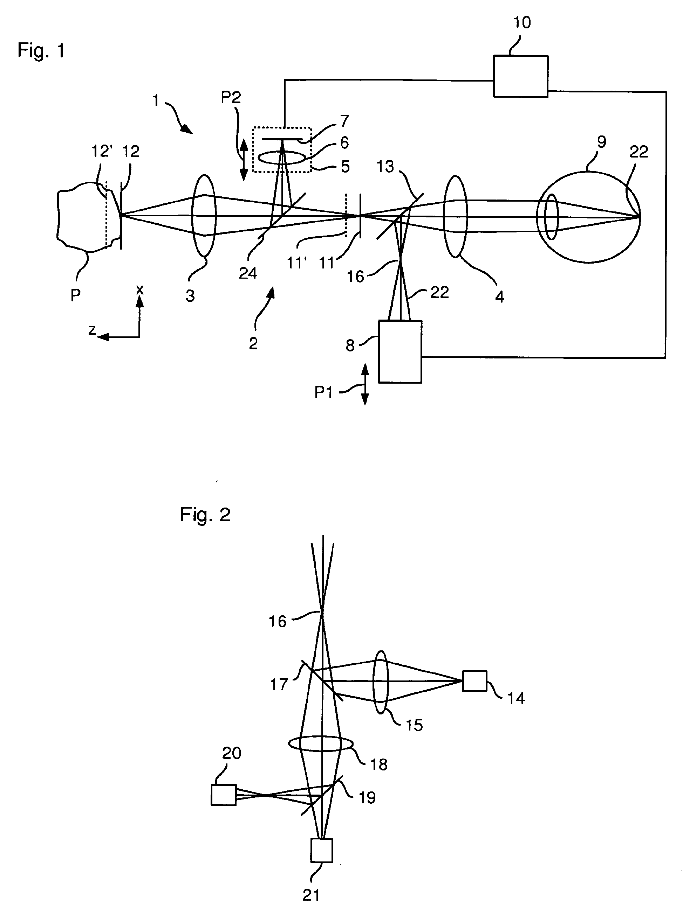

Bei der in Fig. 1 gezeigten Ausführungsform ist die erfindungsgemäße Abbildungsvorrichtung 1 als monookulares Mikroskop mit einer Fotodokumentation ausgebildet und umfaßt ein Beobachtungsmodul 2 mit einem Objektiv 3 und einem Okular 4, ein Bildaufnahmemodul 5 mit einer ersten Optik 6 und einem Bildsensor 7, ein Meßmodul 8 zum Messen des Akkommodationszustandes eines Auges 9 eines Benutzers sowie eine Steuereinheit 10.

Mit dem Beobachtungsmodul 2 kann der Benutzer eine Probe bzw. ein Objekt P vergrößert wahrnehmen, wobei das Objektiv 3 die Probe P in eine Zwischenbildebene 11 abbildet und der Benutzer die in die Zwischenbildebene 11 abgebildete Probe durch das Okular 4 wahrnehmen kann.

Wenn der Benutzer die Probe P scharf wahrnimmt, weist das Auge 9 einen Akkommodationszustand auf, mit dem die in die Zwischenbildebene 11 abgebildete Probe P scharf wahrgenommen wird. Durch den Akkommodationszustand des Auges 9 und die optischen Eigenschaften von Objektiv 3 und Okular 4 wird somit eine Fokusebene 12 festgelegt.

Wenn der Benutzer den Fixaktionszustand bzw. den Akkommodationszustand des Auges 9 ändert, wird durch das Okular 4 eine andere Zwischenbildebene 11' scharf wahrgenommen bzw. scharf auf die Retina des Auges 9 abgebildet. Dies entspricht dann einer in z-Richtung versetzten Fokusebene 12'. In dieser Art und Weise kann der Benutzer durch die Änderung der Akkommodation seines Auges 9 in der abgebildeten Probe in z-Richtung die Lage der Fokusebene 12 verschieben.

Das Bildaufnahmemodul 5 ist über einen Teiler 24 in den Beobachtungsstrahlengang des Beobachtungsmoduls 2 eingekoppelt und so ausgebildet, das es die Fokusebene 12 scharf auf den Bildsensor 7 abbildet. Um nun sicherzustellen, daß mit dem Bildaufnahmemodul 5 stets die tatsächliche Fokusebene 12, 12' scharf aufgenommen wird, auf die der Benutzer mit seinem Auge 9 fokussiert, wird mittels dem Meßmodul 8 der Akkommodationszustand des Auges 9 laufend ermittelt und wird in Abhängigkeit des gemessenen Akkommodationszustandes die Lage der Fokusebene des Bildaufnahmemoduls 5 so geändert bzw. eingestellt, daß sie mit der Fokusebene 12, 12' zusammenfällt.

Zum Messen des Akkommodationszustandes des Auges 9 wird ein Meßstrahlengang 22 über einen Teiler 13 in den Beobachtungsstrahlengang des Beobachtungsmoduls 2 und somit in das Auge 9 eingekoppelt. In Fig. 2 ist eine Ausgestaltung des Meßmoduls 8 gezeigt. Das Meßmodul 8 enthält eine Punktlichtquelle 14, wie z.B. eine LED, eine Laserquelle, insbesondere ein VCSEL, eine SLD (Superluminiszenz-Diode), etc. Die Punktlichtquelle 14 wird mittels einer ersten Meßmoduloptik 15 in eine Ebene 16 abgebildet, die konjugiert ist zur Zwischenbildebene 11. Da der Meßstrahlengang 22 über den Teiler 13 in den Beobachtungsstrahlengang eingekoppelt wird, wird die Punktlichtquelle 14 fokussiert auf die Retinaebene 22 des Auges 9 abgebildet.

Das von der Retina des Auges 9 zurückgestreute Licht durchläuft den Strahlengang in umgekehrter Richtung und gelangt durch einen zweiten Teiler 17 zu einer zweiten Meßmoduloptik 18, die eine Abbildung in eine weitere Zwischenbildebene durchführt. Zwischen der zweiten Meßmoduloptik 18 und der weiteren Zwischenbildebene steht ein dritter Teiler 19, der das Licht zu etwa gleichen Teilen auf zwei Detektoren 20, 21 abbildet, wobei der Detektor 20 kurz hinter der weiteren Zwischenbildebene und der Detektor 21 kurz vor der weiteren Zwischenbildebene angeordnet ist.

Das Intensitätssignal der beiden Detektoren 20, 21 wird der Steuereinheit 10 zugeführt, die aus diesem Regelsignale für das Bildaufnahmemodul 5 sowie für das Meßmodul 8 erzeugt. Die Regelsignale dienen dazu, das Meßmodul 8 in x-Richtung (Doppelpfeil P1) solange zu verschieben, bis die Differenz der Signale der beiden Detektoren 20, 21 Null wird. Wenn das Differenzsignal Null ist, ist die Ebene 16 konjugiert zu der Zwischenbildebene 11, 11', auf die der Benutzer fokussiert.

Entsprechend der Verschiebung des Meßmoduls 8 wird das Bildaufnahmemodul 5 in x-Richtung (Doppelpfeil P2) verschoben, um die Fokusebene des Bildaufnahmemoduls 5 so zu legen, daß sie mit der Fokusebene 12, 12' zusammenfällt, die der Benutzer über das Beobachtungsmodul scharf wahrnimmt. Damit wird sichergestellt, daß das Bildaufnahmemodul 5 stets die Fokusebene 12, 12' aufnimmt, auf die der Benutzer fokussiert.

Bei der in Fig. 2 gezeigten Ausbildung des Meßmoduls 8 können alle Arten von konfokalen Sensoren für die Detektoren 20 und 21 verwendet werden. Es ist auch möglich, nur einen einzigen Detektor zur Messung vorzusehen. In diesem Fall kann auf den dritten Teiler 19 verzichtet werden und der Detektor (z.B. Detektor 21) muß über den Fokuspunkt hinaus bewegt werden. Die Position des höchsten Signals am Detektor 21 ist dann ein Maß für die gesuchte Fokusposition. Ein solcher Sensor ist technisch einfacher aufgebaut als die konfokalen Sensoren 20, 21 gemäß Fig. 2.

Die konfokalen Detektoren 20 und 21 können z.B. so ausgebildet sein, wie gemäß Fig. 2 der DE 10 2005 022 125 A1. Die entsprechende Beschreibung in der DE 10 2005 022 125 A1 wird hiermit durch Bezugnahme aufgenommen. Bei diesen Sensoren kann jede Position innerhalb eines weiten Fangebereichs um den Fokuspunkt mit hoher Genauigkeit vermessen und gehalten werden. Solche Sensoren würden in einem nicht-verschiebbaren Meßmodul 8 verwendet werden und ihr Fokusabweichungssignal kann direkt zur Steuerung der Verschiebung des Bildaufnahmemoduls 5 verwendet werden.

Eine weitere Möglichkeit den Akkommodationszustand des Auges 9 zu messen, stellen Wellenfrontsensoren dar. Diese benutzen eine Beleuchtungsquelle ähnlich zu den konfokalen Sensoren und beleuchtungsseitig auch den gleichen Strahlengang wie in Fig. 2. Detektionsseitig wird aber das Licht mit einem Shack-Hartmann-Sensor detektiert, der anstelle der Elemente 18 - 21 einen ortsauflösenden Sensor mit in der Pupille/Apertur geteilten Optiken (z.B. Mikrolinsenarray) aufweist. Auf dem Detektor entsteht dann ein Punktmuster und es kann aus dem Abstand der Punkte auf dem Detektor die Wellenfrontform des Meßlichtes beim Austritt aus dem Auge 9 berechnet werden. Aus dieser Wellenfrontform läßt sich dann der Akkommodationszustand bzw. der Refraktionswert des Auges 9 ableiten.

Da für die hier beschriebene Anwendung nicht die genaue Form der Wellenfront, sondern nur die Krümmung der Wellenfront (= Refraktionswert) des Auges 9 gemessen werden soll, genügen deutlich vereinfachte Shack-Hartmann-Sensoren mit wenigen Subaperturen (z.B. zwei, vier oder sechs).

Bei den bisher beschriebenen Ausführungsformen wurde beim Bildaufnahmemodul 5 die erste Optik 6 in x-Richtung verschoben. Es ist jedoch auch möglich, variable optische Elemente vorzusehen, deren Brechkraft veränderbar ist, um die Lage der Fokusebene des Bildaufnahmemoduls 5 zu ändern. Auch zueinander bewegte Freiformelemente können benutzt werden.

Die Fokuslage bzw. der Akkommodationszustand des Auges 9 wird bevorzugt im IR-Wellenlängenbereich bestimmt.

Bei der bisherigen Beschreibung wurde von einer monookularen Ausbildung der Abbildungsvorrichtung 1 ausgegangen. Natürlich kann die Abbildungsvorrichtung 1 auch binokular ausgebildet werden und für jedes der beiden Augen die beschriebene Anpassung durchgeführt werden. Ferner kann bei der Bestimmung der Akkommodation von beiden Augen ein automatischer Dioptrienausgleich zwischen den Augen vorgenommen werden.

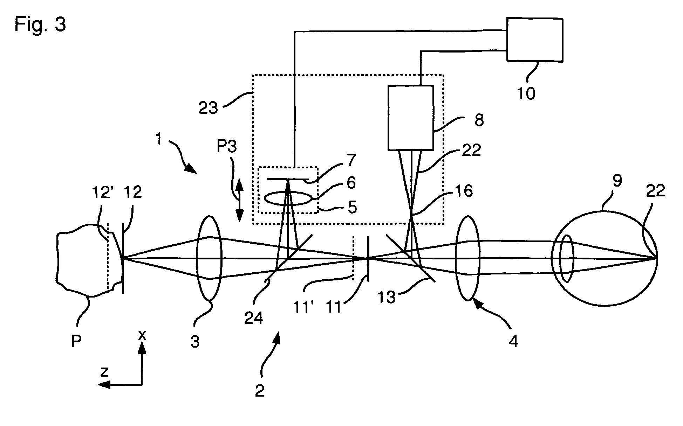

In Fig. 3 ist eine Abwandlung der Ausführungsform gemäß Fig. 1 gezeigt, wobei gleiche Elemente mit gleichen Bezugszeichen bezeichnet sind und zu deren Beschreibung auf die obigen Ausführungen verwiesen wird. Bei der Ausführungsform gemäß Fig. 3 sind das Bildaufnahmemodul 5 und das Meßmodul 8 so miteinander mechanisch gekoppelt, daß sie nur gleichzeitig in x-Richtung bewegt werden können (Doppelpfeil P3). Es muß nur ein Aktuator (hier z.B. ein Schrittmotor) vorgesehen werden, um die beiden Module 5, 8 zu bewegen. Dies ist durch die Punktlinie 23 angedeutet.

Bei den bisher beschriebenen Ausführungsformen sind die Strahlteiler 13 und 24 jeweils in einen Teil des Beobachtungsstrahlenganges angeordnet, in dem die Strahlung divergiert bzw. konvergiert. Natürlich ist es auch möglich, die Strahlteiler 13 und 24 in einem Teil des Beobachtungsstrahlenganges anzuordnen, in dem die Strahlung kollimiert ist. Auch ist es möglich, einen gemeinsamen Strahlteiler für das Bildaufnahmemodul 5 und das Meßmodul 8 im Beobachtungsstrahlengang vorzusehen. Da das Meßmodul 8 Infrarotstrahlung verwendet und das Bildaufnahmemodul 5 Strahlung aus dem sichtbaren Wellenlängenbereich aufnimmt, ist es dann vorteilhaft, einen zusätzlichen Absorptionsfilter vor dem Bildsensor 7 anzuordnen.

Das Meßmodul 8 kann so ausgebildet sein, daß das von der Punktlichtquelle 14 stammende und auf die Retinaebene 22 des Auges 9 fokussierte Licht polarisiert ist (z.B. linear polarisiert). Die Detektion des zurückgestreuten Lichtes mittels der Detektoren 20 und 21 wird dann linear senkrecht zur Eingangspolarisation polarisiert detektiert. In dieser Art und Weise kann quasi eine polarisationsgekreuzte Detektion durchgeführt werden, so daß unerwünschtes Streulicht das von anderen Grenzflächen und nicht von der Retina reflektiert wird, unterdrückt wird. Beispielsweise kann Streulicht von der Kornea und vom Okular 4 unterdrückt werden. Das von der Retina reflektierte Licht kann detektiert werden, weil die Polarisation des Lichtes beim Durchgang durch die Kornea und bei der Streuung an der Retina verändert und damit zum Teil durch den gekreuzten Detektionspolarisator transmittiert und detektiert werden kann. Damit kann die Genauigkeit des Verfahrens gesteigert werden.

Technisch kann dies in der Art und Weise durchgeführt werden, daß ein entsprechender Polarisator vor der Punktlichtquelle 14 und ein entsprechender Polarisator vor den beiden Detektoren 20 und 21 positioniert ist. Insbesondere kann z.B. der Strahlteiler 17 als polarisierender Strahlteiler ausgeführt werden. Natürlich muß nicht lineare Polarisation verwendet werden. Es können auch andere, zueinander orthogonale Polarisationszustände genutzt werden.

Des weiteren ist es möglich, bei dem Meßmodul 8 statt der beiden Detektoren 20 und 21 und dem Strahlteiler 19 einen einzelnen ortsauflösenden Detektor vorzusehen, wobei die gemessene Spotgröße (bzw. die Größe des Zerstreuungskreises) gemessen werden kann. Natürlich kann zusätzlich auch noch die Intensität gemessen werden. Die Meßsignale werden dann in der beschriebenen Art und Weise der Steuereinheit 6 zugeführt.

Der ortsauflösende Detektor kann als ortsauflösender CMOS- oder CCD-Sensor oder auch als flächenseparierter Sensor mit zwei oder mehr einzeln auslösbaren Teilflächen ausgebildet sein. Es sind auch Anordnungen mit außeraxialen Quadrantendioden oder PSD oder auch Diodenarrays möglich.

## Patentansprüche

1. Anzeigevorrichtung mit
einem einen Bildsensor (7) und eine erste Fokusebene aufweisenden Bildaufnahmemodul (5), das ein Objekt (P) aufnimmt,
einem Beobachtungsmodul (2), das das Objekt (P) so abbildet, daß es ein Benutzer mit einem Auge (9) wahrnehmen kann,
wobei durch das Beobachtungsmodul (2) und den Akkommodationszustand des Auges (9) eine zweite Fokusebene festgelegt ist,
**dadurch gekennzeichnet, daß**
ein Meßmodul (8) zum Messen des Akkommodationszustandes des Auges (9) und
eine Steuereinheit (10) vorgesehen sind, wobei die Steuereinheit (10) in Abhängigkeit des gemessenen Akkommodationszustandes die Lage der ersten Fokusebene so einstellt, daß sie mit der zweiten Fokusebene zusammenfällt.

2. Anzeigevorrichtung nach Anspruch 1, **dadurch gekennzeichnet, daß** das Bildaufnahmemodul (5) eine erste Optik (6) zur Abbildung des Objektes (P) auf den Bildsensor (7) aufweist.

3. Anzeigevorrichtung nach Anspruch 2, **dadurch gekennzeichnet, daß** die Brechkraft der ersten Optik (6) änderbar ist, um die Lage der ersten Fokusebene einzustellen.

4. Anzeigevorrichtung nach Anspruch 2 oder 3, **dadurch gekennzeichnet, daß** der Abstand zwischen der ersten Optik (6) und dem Bildsensor (7) änderbar ist, um die Lage der ersten Fokusebene einzustellen.

5. Anzeigevorrichtung nach einem der obigen Ansprüche, **dadurch gekennzeichnet, daß** das Bildaufnahmemodul zum Aufnehmen des Objektes zumindest einen Teil des Beobachtungsmoduls nutzt.

6. Anzeigevorrichtung nach einem der obigen Ansprüche, **dadurch gekennzeichnet, daß** das Meßmodul (8) den Akkommodationszustand des Auges (9) konfokal mißt.

7. Anzeigevorrichtung nach einem der obigen Ansprüche, **dadurch gekennzeichnet, daß** das Meßmodul (8) zum Messen des Akkommodationszustandes des Auges (9) Infrarotstrahlung verwendet.

8. Anzeigevorrichtung nach einem der obigen Ansprüche, **dadurch gekennzeichnet, daß** das Meßmodul (8) den Akkommodationszustand des Auges laufend mißt.

9. Anzeigevorrichtung nach einem der obigen Ansprüche, **dadurch gekennzeichnet, daß** das Meßmodul (8) eine Lichtquelle (14), die einen Lichtstrahl abgibt, ein optisches System (15, 18), das den Lichtstrahl über das Beobachtungsmodul (2) in das Auge des Benutzers führt, und einen Detektor (20, 21) aufweist,
wobei der am Augenhintergrund des Auges reflektierte Lichtstrahl über das Beobachtungsmodul (2) und das optische System (18) auf den Detektor (20, 21) gerichtet wird, der ein Detektorsignal abgibt, das zur Messung des Akkommodationszustandes ausgewertet wird.

10. Anzeigevorrichtung nach einem der obigen Ansprüche, **dadurch gekennzeichnet, daß** das Bildaufnahmemodul (2) und das Meßmodul (8) gleichzeitig bewegt werden, wobei durch die Bewegung des Meßmoduls (8) der Akkommodationszustand des Auges gemessen wird und durch die Bewegung des Bildaufnahmemoduls (2) die Lage der ersten Fokusebene so eingestellt wird, daß sie mit der zweiten Fokusebene zusammenfällt.

11. Anzeigevorrichtung nach Anspruch 10, **dadurch gekennzeichnet, daß** das Bildaufnahmemodul (2) und das Meßmodul (8) mechanisch miteinander gekoppelt sind, um die gleichzeitige Bewegung durchführen zu können.

12. Anzeigeverfahren, bei dem
mit einem einen Bildsensor und eine Fokusebene aufweisenden Bildaufnahmemodul ein Objekt aufgenommen wird, und
mit einem Beobachtungsmodul das Objekt so abgebildet wird, daß es ein Benutzer mit einem Auge wahrnehmen kann,
wobei durch das Beobachtungsmodul und den Akkommodationszustand des Auges eine zweite Fokusebene festgelegt ist,
**dadurch gekennzeichnet, daß** der Akkommodationszustand des Auges gemessen und in Abhängigkeit des gemessenen Akkommodationszustandes die Lage der ersten Fokusebene so eingestellt wird, daß sie mit der zweiten Fokusebene zusammenfällt.

## Claims

1. Display device with
an image acquisition module (5), having an image sensor (7) and a first focal plane, which captures a picture of an object (P),
an observation module (2) which images the object (P) such that a user can perceive it with his eye (9),
wherein a second focal plane is set by the observation module (2) and the accommodation state of the eye (9),
**characterized in that**
a measuring module (8) for measuring the accommodation state of the eye (9) and
a control unit (10) which adjusts the position of the first focal plane on the basis of the measured accommodation state such that it coincides with the second focal plane are provided.

2. Display device according to claim 1, **characterized in that** the image acquisition module (5) has a first lens system (6) for imaging the object (P) onto the image sensor (7).

3. Display device according to claim 2, **characterized in that** the refractive power of the first lens system (6) is alterable in order to adjust the position of the first focal plane.

4. Display device according to claim 2 or 3, **characterized in that** the distance between the first lens system (6) and the image sensor (7) is alterable in order to adjust the position of the first focal plane.

5. Display device according to one of the above claims, **characterized in that** the image acquisition module uses at least part of the observation module to capture a picture of the object.

6. Display device according to one of the above claims, **characterized in that** the measuring module (8) measures the accommodation state of the eye (9) confocally.

7. Display device according to one of the above claims, **characterized in that** the measuring module (8) uses infrared radiation to measure the accommodation state of the eye (9).

8. Display device according to one of the above claims, **characterized in that** the measuring module (8) continuously measures the accommodation state of the eye.

9. Display device according to one of the above claims, **characterized in that** the measuring module (8) has a light source (14) which emits a light beam, an optical system (15, 18) which guides the light beam into the eye of the user via the observation module (2), and a detector (20, 21),
wherein the light beam reflected at the fundus of the eye is directed via the observation module (2) and the optical system (18) onto the detector (20, 21), which emits a detector signal which is evaluated to measure the accommodation state.

10. Display device according to one of the above claims, **characterized in that** the image acquisition module (2) and the measuring module (8) are moved at the same time, wherein the accommodation state of the eye is measured through the movement of the measuring module (8) and the position of the first focal plane is adjusted through the movement of the image acquisition module (2) such that it coincides with the second focal plane.

11. Display device according to claim 10, **characterized in that** the image acquisition module (2) and the measuring module (8) are coupled together mechanically in order to be able to carry out the simultaneous movement.

12. Display method, in which
a picture of an object is captured with an image acquisition module having an image sensor and a focal plane, and
the object is imaged with an observation module such that a user can perceive it with his eye, wherein a second focal plane is set by the observation module and the accommodation state of the eye,
**characterized in that** the accommodation state of the eye is measured and the position of the first focal plane is adjusted on the basis of the measured accommodation state such that it coincides with the second focal plane.

## Revendications

1. Dispositif d'affichage comportant
un module de prise de vue (5), qui comporte un capteur d'image (7) et un premier plan focal et qui photographie un objet (P),
un module d'observation (2) qui représente l'objet (P) de telle sorte qu'un utilisateur peut le percevoir avec un oeil (9),
un deuxième plan focal étant défini par le module d'observation (2) et l'état de l'accommodation de l'oeil (9),
**caractérisé en ce que**
il est prévu un module de mesure (8) destiné à mesurer l'état de l'accommodation de l'oeil (9) et
une unité de commande (10), ladite unité de commande (10) réglant, en fonction de l'état de l'accommodation mesuré, la position du premier plan focal, de telle sorte que celle-ci coïncide avec celle du deuxième plan focal.

2. Dispositif d'affichage selon la revendication 1, **caractérisé en ce que** le module de prise de vue (5) comporte une première optique (6) pour représenter l'objet (P) sur le capteur d'image (7).

3. Dispositif d'affichage selon la revendication 2, **caractérisé en ce que** la vergence de la première optique (6) est modifiable pour régler la position du premier plan focal.

4. Dispositif d'affichage selon la revendication 2 ou 3, **caractérisé en ce que** la distance entre la première optique (6) et le capteur d'image (7) est modifiable pour régler la position du premier plan focal.

5. Dispositif d'affichage selon l'une quelconque des revendications précédentes, **caractérisé en ce que** le module de prise de vue utilise au moins une partie du module d'observation pour photographier l'objet.

6. Dispositif d'affichage selon l'une quelconque des revendications précédentes, **caractérisé en ce que** le module de mesure (8) effectue une mesure confocale de l'état de l'accommodation de l'oeil (9).

7. Dispositif d'affichage selon l'une quelconque des revendications précédentes, **caractérisé en ce que** le module de mesure (8) utilise un rayonnement infrarouge pour mesurer l'état de l'accommodation de l'oeil (9).

8. Dispositif d'affichage selon l'une quelconque des revendications précédentes, **caractérisé en ce que** le module de mesure (8) mesure en continu l'état de l'accommodation de l'oeil.

9. Dispositif d'affichage selon l'une quelconque des revendications précédentes, **caractérisé en ce que** le module de mesure (8) comporte une source lumineuse (14), qui émet un rayon lumineux, un système optique (15, 18), qui guide le rayon lumineux vers l'oeil de l'utilisateur en passant par le module d'observation (2), et un détecteur (20, 21),
le rayon lumineux réfléchi sur le fond de l'oeil étant dirigé, en passant par le module d'observation (2) et le système optique (18), sur le détecteur (20, 21), qui délivre un signal de détection exploité pour mesurer l'état de l'accommodation.

10. Dispositif d'affichage selon l'une quelconque des revendications précédentes, **caractérisé en ce que** le module de prise de vue (2) et le module de mesure (8) sont déplacés en même temps, le mouvement du module de mesure (8) permettant de mesurer l'état de l'accommodation de l'oeil, et le mouvement du module de prise de vue (2) permettant de régler la position du premier plan focal de telle sorte que celle-ci coïncide avec celle du deuxième plan focal.

11. Dispositif d'affichage selon la revendication 10, **caractérisé en ce que** le module de prise de vue (2) et le module de mesure (8) sont couplés mécaniquement l'un à l'autre pour pouvoir effectuer le mouvement simultané.

12. Procédé d'affichage, dans lequel
un objet est photographié au moyen d'un module de prise de vue comportant un capteur d'image et un plan focal, et
l'objet étant représenté avec un module d'observation de telle sorte qu'un utilisateur peut le percevoir avec un oeil,
un deuxième plan focal étant défini par le module d'observation et l'état de l'accommodation de l'oeil,
**caractérisé en ce que** l'état de l'accommodation de l'oeil est mesuré et, en fonction de l'état de l'accommodation mesuré, la position du premier plan focal est réglée de telle sorte que celle-ci coïncide avec celle du deuxième plan focal.
